# EUROPÄISCHE PATENTSCHRIFT

(11) **EP 1 752 454 B1**
(45) Veröffentlichungstag und Bekanntmachung des Hinweises auf die Patenterteilung: **07.05.2008**
(21) Anmeldenummer: 06117876.0
(22) Anmeldetag: 26.07.2006
(51) Int. Cl.: C07D 295/00

(54) **Vefahren zur destillativen Abtrennung von Piperazin aus einem Ethylendiamin-Piperazin-Gemisch**
Method for separating piperazine from a mixture of ethylenediamine-piperazine through destillation
Procédé de séparation de piperazine d`un mélange de ethhylenediamine-piperazine par distillation

(30) Priorität: 13.08.2005 DE 102005038376
(43) Veröffentlichungstag der Anmeldung: 14.02.2007
(73) Patentinhaber: BASF SE, 67056 Ludwigshafen (DE)
(72) Erfinder: Jödecke, Michael, 67240, Bobenheim-Roxheim (DE); Lang, Ortmund, 66909, Quirnbach (DE); Cauwenberge, Gunther, 9140, Temse (BE); Frauenkron, Matthias, 2920, Kalmhout (NL)

(56) Entgegenhaltungen:
- GB-A- 1 263 588
- US-A- 3 105 019

## Beschreibung

Die Erfindung betrifft ein Verfahren zur kontinuierlichen, destillativen Abtrennung von Piperazin aus einem Ethylendiamin-Piperazin-Gemisch unter Druck und bei erhöhter Temperatur, wobei das Ethylendiamin am Kopf und das Piperazin am Sumpf einer Destillationskolonne ausgetragen werden.

Piperazin findet Anwendung bei der Herstellung von Pharmazeutika für die Human- und Veterinärmedizin, Kosmetika und Photochemikalien. Ein wichtiges Qualitätsmerkmal des Piperazins ist dabei neben seiner meist gaschromatographisch ermittelten Reinheit der Grad der Verfärbung und die Farbstabiliät.

Als ein Maß für den Grad der Verfärbung wird meist die so genannte Farbzahl herangezogen. Die Farbzahl ist ein unter festgelegten Bedingungen ermittelter Kennwert für die Farbe von transparenten Substanzen, der durch optischen Vergleich festgestellt wird. Eine häufig verwendete Farbzahl bei Flüssigkeiten ist die APHA (American Public Health Association)-Farbzahl (Römpp Chemie Lexikon 1995).

Nach bekannten Verfahren erfolgt der Erhalt von Piperazin in gewünschter Reinheit durch destillative Abtrennung von einem Produktgemisch. Hierbei stehen mittlerweile derart leistungsstarke Destillationsapparaturen zur Verfügung, dass die letztendlich erhaltene Piperazinfraktion bereits den gewünschten Reinheitsgrad besitzt. Somit beinhaltet die Destillation sowohl den Verfahrensschritt der Abtrennung (Isolierung) als auch den der Reinigung in einem. Dies stellt aus ökonomischer Sicht ein besonders effizientes Vorgehen dar, auch wenn das Piperazin erst mehrfach in einer oder mehreren Kolonnen fraktioniert werden muss.

Im technischen Maßstab wird Piperazin meist bei der Herstellung verschiedener Ethylenamine als eines der Wertprodukte erhalten. Hierbei beruht die Synthese auf der Umsetzung von Ethylendichlorid (EDC-Verfahren) oder Monoethanolamin (MEOA-Verfahren) mit Ammoniak. Weitere Koppelprodukte dieser Umsetzung sind Ethylendiamin (EDA), Diethylentriamin (DETA), Triethylentetramin (TETA) und höhere lineare und cyclische Ethylenamine sowie zusätzlich Aminoethylethanolamin (AEEA) beim MEOA Verfahren.

Beide Syntheserouten basieren letztlich auf der Oxidation von Ethylen (mit Chlor oder Sauerstoff) zu EDC bzw. Ethylenoxid (EO) und anschließender ein- oder zweistufiger Umsetzung mit Ammoniak. Zwar beinhaltet das MEOA Verfahren damit einen zusätzlichen Syntheseschritt, das Oxidationsmittel Chlor ist jedoch teuer und zusätzlich ist ein Salzanfall unvermeidlich.

Im Vergleich zum EDC Verfahren gewährleistet das MEOA-Verfahren einen erhöhten Anteil an cyclischen Verbindungen, so dass dieses, wenn ein piperazinhaltiger Produktmix gewünscht wird, das bevorzugte Verfahren darstellt.

Unabhängig von der Wahl des Verfahrens erfolgt die Aufreinigung und Trennung des Ethylenamin-Produktmixes bei industrieller Produktion meist über eine Kaskade von Kolonnen in kontinuierlichem Betrieb. Zunächst wird dabei der Ammoniak in einer Druckkolonne abgezogen, danach erfolgt die Abdestillation des gebildeten (oder beim EDC Verfahren zugesetzten) Prozesswassers.

GB 1 263 588 beschreibt hierbei die Reinigung des Produktmixes durch das azeotrope Entfernen von Verunreinigungen beim Abdestillieren von Wasser.

US 3,105,019 beschreibt hingegen den Zusatz organischer Lösemittel als Schleppmittel für Piperazin in der Destillation speziell zur Abtrennung einer Piperazin-TEDA Fraktion.

Alle oben beschriebenen Verfahren liefern zwar Piperazin oder andere Ethylamin-Produkte oder -Produktgemische mit ausreichender Reinheit, jedoch ist nicht gewährleistet, dass die Produkte über eine ausreichende Farbstabilität verfügen, selbst wenn die ermittelte Farbzahl unmittelbar nach Isolierung den Anforderungen entspricht.

Dies liegt daran, dass die Verfärbungskomponenten in der Regel als Einzelverbindungen nicht fassbar sind und beispielsweise in der gaschromatographischen Reinheitskontrolle unter der Detektionsgrenze liegen. Insbesondere im MEOA-Verfahren basieren diese Verbindungen auf Acetaldehydfragmenten und deren kondensierten Folgeprodukten. Hierbei bilden sich die Chromophor-gebenden Verbindungen erst im Laufe der Zeit, so dass eine Farbzahländerung häufig erst nach Lagerung dahingehend eintritt, dass die gewünschte Spezifikation nicht mehr erreicht wird.

Diese Verunreinigungen tauchen insbesondere im MEOA Verfahren auf, da die Aminierung von MEOA auf Ethyleneinheiten basiert, die die Bildung von C2-Fragmenten durch Dehydratisierung und Desaminierung unter den Reaktionsbedingungen ermöglichen. Diese Fragmente, speziell Acetaldehyd und höher kondensierte Folgeprodukte, verursachen hierbei eine nachhaltige Verschlechterung der Produktqualität, insbesondere der Farbstabilität. Auch hierbei gilt, dass in den isolierten Komponenten nach der Destillation diese Verunreinigungen jedoch aufgrund ihrer Inhomogenität und geringen Konzentration in der Regel analytisch nicht nachzuweisen sind.

Es war daher Aufgabe der Erfindung, ein verbessertes, effizientes und wirtschaftliches Verfahren zur Herstellung von reinem Piperazin mit verbesserter Qualität bezüglich Farbe, Farbstabilität und Geruch zu schaffen.

Zur Lösung dieser Aufgabe werden die Maßnahmen nach dem Kennzeichen des Patentanspruchs vorgeschlagen.

Die Erfindung verwendet ein Ethylendiamin-Piperazin-Gemisch, welches in an sich bekannter Weise erhalten wird. Das Gemisch wird in einer Destillationskolonne unter Druck und bei erhöhter Temperatur aufgetrennt. Hierfür eignen sich übliche Destillationsvorrichtungen mit Verdampfer und Kondensator. Es wird bevorzugt eine Destillationskolonne mit im Allgemeinen 10 bis 60, zweckmäßig 30 bis 40 theoretischen Trennstufen in Form von Böden, geordneten Packungen oder Füllkörpern eingesetzt. Als Böden kommen Glockenböden, Siebböden, Ventilböden, Thormannböden, Streuberböden oder Dual-Flow-Böden in Betracht. Bevorzugte Packungen sind kompakte Flächengebildet oder Gewebe aus Metall oder Kunststoff. Vorteilhafte Füllkörper sind Ringe, wie Raschig-, Intos- oder Pallringe, Sattelkörper, wie Barrel- oder Intaloxsättel sowie Top-Pak oder Geflechte.

Der Zulauf des Ethylendiamin-Piperazin-Gemisches in die Destillatinskolonne erfolgt im Bereich der theoretischen Trennstufen 5 bis 20, insbesondere 10 bis 15. Die Destillation wird im Druckbereich von 0,5 bis 2 bar, vorzugsweise 0,8 bis 1,5 bar und bei einer Temperatur im Sumpf der Kolonne von etwa 140 bis etwa 170°C durchgeführt.

Aufgrund der Temperaturempfindlichkeit des Ethylendiamin-Piperazin-Gemisches ist es zweckmäßig, die Destillationskolonne mit einem Verdampfer zu betreiben, der eine geringe Wandtemperatur sowie einen kleinen Flüssigkeitsinhalt aufweist. Insgesamt hat es sich als besonders günstig erwiesen, einen Fallfilm- oder Dünnschichtverdampfer zu verwenden. Zur schonenden Eindampfung empfiehlt sich eine Temperaturdifferenz zwischen der Dampf- und der Produktseite von ≦ 30°C, insbesondere ≦ 20°C. Der Kolonnensumpf und der Verdampfersumpf werden dabei so gestaltet, dass die Verweilzeit des Gemischs weniger als 60 Minuten beträgt.

Das durch Destillation erhaltene Ethylendiamin wird am Kopf der Kolonne und das Piperazin am Sumpf der Kolonne ausgetragen. Dabei wird das Ethylendiamin als Rücklauf wieder in die Destillationskolonne eingespeist, wobei diese so eingestellt wird, dass das Rücklaufverhältnis 0,4 bis 0,8 beträgt. Das Piperazin wird erfindungsgemäß unmittelbar einer Kreisförderung unterworfen, dabei durch eine bei einer Temperatur von etwa 160°C bis etwa 170°C betriebenen Verdampfereinheit gefördert und in die Destillationskolonne zurückgeführt. Als Verdampfereinheit eignen sich Fallfilmverdampfer, Dünnschichtverdampfer, Kurzwegverdampfer oder Wendelrohrverdampfer. Nach einer Verweilzeit von etwa 30 min. bis etwa 60 min in der Kreisförderung wird das Piperazin dampfförmig aus einem Seitenabzug im unteren Bereich der Destillationskolonne kontinuierlich ausgeschleust.

Das dampfförmige Piperazin aus dem Seitenabzug kann anschließend mit einem herkömmlichen Kondensator oder mit einem Piperazin-Quench mit außenliegendem Kühler kondensiert werden und in Form von Chips oder in anderer Form gewonnen und gelagert werden.

Das Piperazin aus dem Seitenabzug hat eine hohe Reinheit (Piperazin-Gehalt > 99,9 Gew.-%) und eine niedrige Farbzahl (< 30 APHA). Es zeichnet sich außerdem durch eine verbesserte Farbzahlstabilität aus. Das Piperazin, welches im Sumpf der Kolonne abgezogen wird, weist ebenfalls eine hohe Reinheit aus. Da es für die Herstellung der wässrigen Piperazin-Lösung verwendet wird, stellt die höhere Farbzahl kein Problem dar.

Die verbesserte Farbstabilität kann im Langzeittest überprüft werden.

Bei dem Farbzahltest erfolgt die Bestimmung der Farbqualität einer Verbindung generell durch Messung der Transmission eingestrahlten Lichtes. Hierzu wird die Schmelze oder eine Lösung einer bestimmten Konzentration in einer Küvette bekannter Schichtdicke mit einem Lichtstrahl einer definierten Wellenlänge durchstrahlt. Der Prozentsatz der durchgelassenen Lichtenergie ergibt bei gegebener Wellenlänge eine definierte Farbzahl. Gebräuchlich für schwachgefärbte Lösungen ist die APHA-Farbskala.

Die Farbzahlmessung erfolgt in einem zuvor auf den mit dem verwendeten Wasser auf den Nullpunkt geeichten Spektrometer in einer 50-mm-Kunststoffküvette. Das Piperazin wird in pulverisierter Form eingewogen, wobei darauf zu achten ist, dass keine übermäßige Erwärmung durch Reibung eintritt. Auch muss der ganze Vorgang zügig in einem gut abgesaugten Ort durchgeführt werden, um Wassereintrag aufgrund der Hygroskopie des Piperazins gering zu halten.

Die Erfindung wird nachfolgend anhand der Beispiele näher erläutert.

### Beispiel 1 (Vergleichsbeispiel, nicht erfindungsgemäß)

Die Versuche wurden mit einer Glockenbodenkolonne DN 50 mit 70 Böden durchgeführt.

Zugeleitet wurden 1500 g/h eines Gemisches aus ca. 90 Gew.-% Ethylendiamin und ca. 10 Gew.-% Piperazin. Die Zuleitung erfolgte auf Boden 26. Das Rücklaufverhältnis betrug ungefähr 1:2. Die Kolonne wurde bei einem Absolutdruck von 1 bar, einer Kopftemperatur von ca. 117°C und einer Sumpftemperatur von ca. 150°C betrieben. Die Eindampfung am Sumpf der Kolonne erfolgte mit einem Umlaufverdampfer. Das Ethylendiamin wurde mit hoher Reinheit am Kopf der Kolonne abgezogen, das Piperazin wurde am Sumpf der Kolonne abgezogen. Das so gewonnene Piperazin hatte eine Reinheit von mindestens 99,9 Gew.-%, wies aber bezüglich seiner Farbe, seiner Farbzahlstabilität und seines Geruches ungenügende Eigenschaften auf und war deshalb nicht marktfähig. Es wurden Farbzahlwerte von 24 APHA unmittelbar nach der Konfektionierung und 65 APHA nach 3 Monaten gemessen.

### Beispiel 2 (erfindungsgemäß)

Bei Durchführung des Versuchs wie in Beispiel 1 beschrieben wurde das am Sumpf der Glockenbodenkolonne ausgetragene Piperazin einer Kreisförderung unterworfen, dabei durch einen bei einer Temperatur von 165°C betriebenen Fallfilmverdampfer gefördert und in die Kolonne zurückgeführt. Die Verweilzeit des Piperazins in der Kreisförderung betrug etwa 40 min. Hierdurch konnte die thermische Belastung des Piperazins im Sumpf der Kolonne reduziert werden. Dampfförmiges Piperazin wurde dann über einen Seitenabzug oberhalb des 4. Bodens der Glockenbodenkolonne kontinuierlich abgezogen, kondensiert und konfektioniert. Das Verhältnis zwischen der Sumpf- und der Seitenabzugsmenge betrug ca. 1:1. Es wurde Piperazin mit einem Reinheitsgrad von > 99,9 Gew.-% erhalten. Die Farbzahl und die Farbzahlstabilität waren im Vergleich zu dem Versuch nach Beispiel 1 deutlich verbessert. Es wurden Farbzahlwerte von 16 APHA unmittelbar nach der Konfektionierung und 42 APHA nach 3 Monaten gemessen.

## Patentansprüche

1. Verfahren zur kontinuierlichen, destillativen Abtrennung von Piperazin aus einem Ethylendiamin-Piperazin-Gemisch unter Druck und bei erhöhter Temperatur, wobei das Ethylendiamin am Kopf und das Piperazin am Sumpf einer Destillationskolonne ausgetragen werden, **dadurch gekennzeichnet, dass** das Piperazin unmittelbar einer Kreisförderung unterworfen, dabei durch eine bei einer Temperatur von etwa 160°C bis etwa 170°C betriebenen Verdampfereinheit gefördert und in die Destillationskolonne zurückgeführt wird und nach einer Verweilzeit von etwa 30 min. bis etwa 60 min. in der Kreisförderung dampfförmig aus einem Seitenabzug im unteren Bereich der Destillationskolonne ausgeschleust wird.

## Claims

1. A process for continuously, distillatively removing piperazine from an ethylenediamine-piperazine mixture under pressure at elevated temperature, by discharging the ethylenediamine at the top and the piperazine at the bottom of a distillation column, which comprises subjecting the piperazine directly to a circulation conveying it through an evaporator unit operated at a temperature of from about 160°C to about 170°C and returning it into the distillation column, and, after a residence time of from 30 min to about 60 min in the circulation system, discharging it in vapor form from a side draw in the lower region of the distillation column.

## Revendications

1. Procédé de séparation en continu par distillation de pipérazine d'un mélange d'éthylènediamine-pipérazine sous pression et à température élevée, l'éthylènediamine étant soutirée à la tête et la pipérazine l'étant au fond d'une colonne de distillation, **caractérisé en ce que** la pipérazine est directement soumise à une conduite circulaire, pendant laquelle elle est conduite par une unité d'évaporation fonctionnant à une température située entre environ 160°C et environ 170°C et recyclée dans la colonne de distillation et après un temps de séjour d'environ 30 minutes jusqu'à environ 60 minutes dans la conduite circulaire, est évacuée sous forme de vapeur dans un soutirage latéral dans la zone inférieure de la colonne de distillation.
